# EUROPEAN PATENT SPECIFICATION

(11) **EP 4 042 947 B1**
(45) Date of publication and mention of the grant of the patent: **19.02.2025**
(21) Application number: 22157078.1
(22) Date of filing: 16.02.2022
(51) Int. Cl.: A61B 8/12, A61B 8/00, A61B 1/00, A61B 1/015, A61B 1/018, A61B 1/05, A61B 1/06, A61B 1/07

(54) **ULTRASOUND ENDOSCOPE**
ULTRASCHALLENDOSKOP
ENDOSCOPE ULTRASONIQUE

(30) Priority: 16.02.2021 JP 2021022185
(43) Date of publication of application: 17.08.2022
(73) Proprietor: FUJIFILM Corporation, Tokyo 106-8620 (JP)
(72) Inventor: KINOMOTO, Noboru, Kanagawa, 258-8538 (JP)
(74) Representative: Dehns Germany Partnerschaft mbB

(56) References cited:
- US-A- 5 842 993
- US-A1- 2008 119 738
- US-A1- 2019 000 417

## Description

### BACKGROUND OF THE INVENTION

### 1. Field of the Invention

The present invention relates to an ultrasound endoscope.

### 2. Description of the Related Art

In recent years, an ultrasound endoscope comprising an observation system that is capable of imaging an image inside a body of a subject, and an ultrasound transducer that is capable of irradiating the inside of the body of the subject with ultrasonic waves and receiving reflected waves to capture video has been used in medical practice. The ultrasound transducer and a signal wire for ultrasonic waves are electrically connected to such an ultrasound endoscope as disclosed in JP4261202B and JP2004-298240A, for example, and in US 2008/119738, US 2019/000417 and US 5, 842, 993.

### SUMMARY OF THE INVENTION

Incidentally, in JP4261202B and JP2004-298240A, in electrically connecting the ultrasound transducer and the signal wire for ultrasonic waves, since the signal wire for ultrasonic waves is made to pass through an opening having a given shape, there is a need to make a cross sectional shape of the signal wire for ultrasonic waves coincide with a cross sectional shape of the opening.

The present invention has been accomplished in view of such a situation, and an object of the present invention is to provide an ultrasound endoscope capable of easily fixing a signal wire bundle including a plurality of coaxial cables.

An ultrasound endoscope of a first aspect is as defined by claim 1.

In an embodiment, in a distal end part cross section taken along a plane that is perpendicular to the central axis of the distal end part and passes through the receiving member and the pressing member, in a case where one area of areas obtained by dividing the distal end part cross section into two with a straight line passing through the central axis is defined as a first divided area, and the other area is defined as a second divided area, the plurality of signal wire bundles are disposed in the first divided area.

In an embodiment, an observation system unit is disposed in the distal end part, and includes an imaging element and a signal cable that is connected to the imaging element, and the signal cable is disposed along at least one of the plurality of signal wire bundles.

In an embodiment, in the distal end part cross section, the plurality of signal wire bundles and the signal cable are disposed in the first divided area.

In an ultrasound endoscope of an embodiment, the pressing member includes a plurality of individual pressing surfaces that individually press the plurality of signal wire bundles for each signal wire bundle, and each individual pressing surface has a curved shape following each signal wire bundle.

In an embodiment, the signal cable is disposed between the plurality of individual pressing surfaces on an opposite side from the plurality of signal wire bundles with the pressing member interposed therebetween.

An ultrasound endoscope of another embodiment further comprises a forceps tube that is disposed in the distal end part along the central axis, and in the distal end part cross section, the plurality of signal wire bundles are disposed in the first divided area, and the forceps tube is disposed in the second divided area.

In an ultrasound endoscope of another embodiment, an observation system unit is disposed in the distal end part, and includes an imaging element and a signal cable that is connected to the imaging element, and in the distal end part cross section, in a case where one area of areas obtained by dividing the distal end part cross section into two with a straight line passing through a central axis of each of the signal cable and the forceps tube is defined as a third divided area, and the other area is defined as a fourth divided area, a part of signal wire bundles among the plurality of signal wire bundles is disposed in the third divided area, and the other part of signal wire bundles is disposed in the fourth divided area.

In an ultrasound endoscope of another embodiment, at a position to be fixed by the receiving member and the pressing member, the plurality of signal wire bundles include an exposed area that is exposed from the outer coat, and an insulating member that has rigidity lower than the outer coat and with which the exposed area is coated.

In an ultrasound endoscope of another embodiment, a locking groove with one end open is provided in one of the receiving member and the pressing member, a locking piece lockable into the locking groove is provided in the other member of the receiving member and the pressing member, and the locking piece is locked into the locking groove, so that relative positions of the receiving member and the pressing member are positioned.

In an ultrasound endoscope of another embodiment, at least one of the receiving member or the pressing member is formed of metal.

In an ultrasound endoscope of another embodiment, the receiving member and the pressing member are insulated from a ground of the distal end part.

In an ultrasound endoscope of another embodiment, an observation system unit is disposed in the distal end part, and includes an imaging element and a signal cable that is connected to the imaging element, and the receiving member and the pressing member are insulated from a ground of the signal cable.

In an ultrasound endoscope of another embodiment, the ultrasound transducer and the plurality of signal wire bundles are electrically connected through a substrate.

In an ultrasound endoscope of another embodiment, the substrate is a flexible print substrate.

With the ultrasound endoscope of the present invention, it is possible to easily fix a signal wire bundle including a plurality of coaxial cables.

### BRIEF DESCRIPTION OF THE DRAWINGS

Fig. 1 is a schematic configuration diagram showing an example of the configuration of an ultrasonography system using an ultrasound endoscope.
Fig. 2 is a partial enlarged perspective view showing the appearance of an example of a distal end part of the ultrasound endoscope shown in Fig. 1.
Fig. 3 is a longitudinal sectional view of the distal end part of the ultrasound endoscope shown in Fig. 2.
Fig. 4 is a sectional view schematically showing the configuration of an example of a coaxial cable.
Fig. 5 is a sectional view schematically showing an example of a signal wire bundle configured with a plurality of coaxial cables.
Fig. 6 is a perspective view showing a part of the distal end part of the ultrasound endoscope.
Fig. 7 is a perspective view showing a part of the distal end part of the ultrasound endoscope including a plurality of signal wire bundles.
Fig. 8 is a perspective view showing a part of the distal end part of the ultrasound endoscope including a plurality of signal wire bundles viewed from a direction different from Fig. 7.
Fig. 9 shows a cross section of the distal end part taken along a plane that is perpendicular to a central axis of the distal end part in which a plurality of signal wire bundles are disposed and passes through a receiving member and a pressing member.
Fig. 10 shows a cross section of the distal end part taken along a plane that is perpendicular to the central axis of the distal end part in which a plurality of signal wire bundles and a signal cable are disposed and passes through the receiving member and the pressing member.
Fig. 11 shows a cross section of the distal end part taken along a plane that is perpendicular to the central axis of the distal end part in which a plurality of signal wire bundles and a forceps tube are disposed and passes through the receiving member and the pressing member.
Fig. 12 shows a cross section of the distal end part taken along a plane that is perpendicular to the central axis of the distal end part in which a plurality of signal wire bundles, the signal cable, and the forceps tube are disposed and passes through the receiving member and the pressing member.
Fig. 13 is a perspective view illustrating a structure for positioning relative positions of the receiving member and the pressing member.
Fig. 14 is a schematic view showing the structures of the distal end part, a bending part, a soft part, and a signal wire bundle.
Fig. 15 is a schematic view showing the structure of the signal wire bundle different from Fig. 14.
Fig. 16 is a diagram illustrating a procedure for electrically connecting the coaxial cable of the signal wire bundle to a flexible print substrate.

### DESCRIPTION OF THE PREFERRED EMBODIMENTS

Hereinafter, a preferred embodiment of an ultrasound endoscope according to the present invention will be described referring to the accompanying drawings.

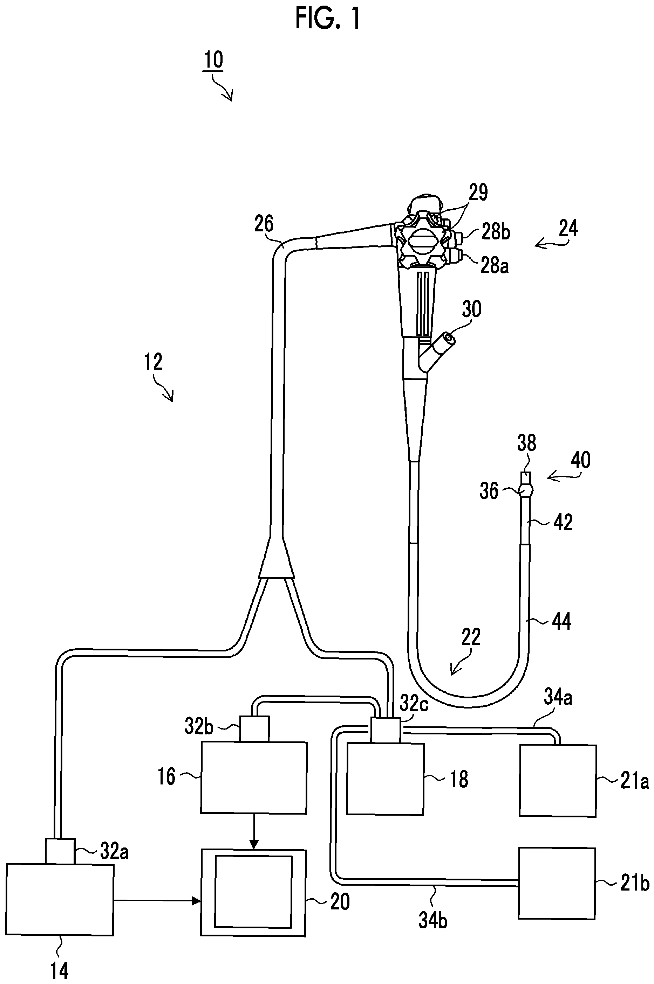

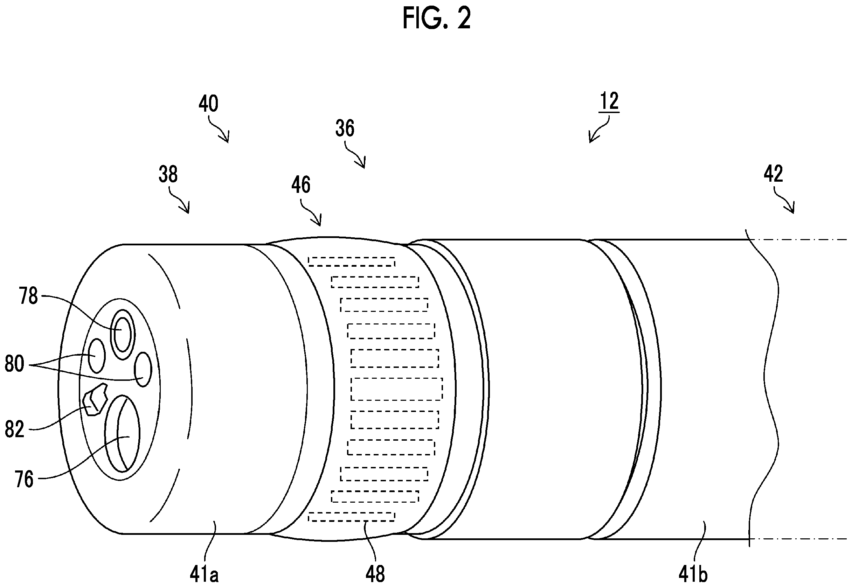

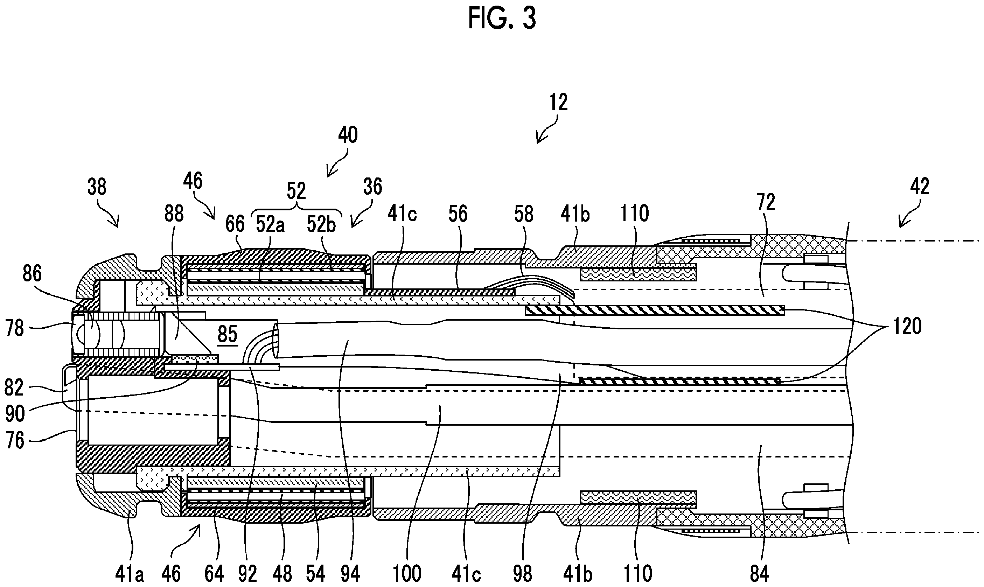

Fig. 1 is a schematic configuration diagram showing an example of an ultrasonography system 10 that uses an ultrasound endoscope 12 of an embodiment. Fig. 2 is a partial enlarged perspective view showing the appearance of an example of a distal end part of the ultrasound endoscope shown in Fig. 1. Fig. 3 is a longitudinal sectional view along a central axis of the distal end part of the ultrasound endoscope shown in Fig. 2.

As shown in Fig. 1, the ultrasonography system 10 comprises the ultrasound endoscope 12, an ultrasound processor device 14 that generates an ultrasound image, an endoscope processor device 16 that generates an endoscope image, a light source device 18 that supplies illumination light, with which the inside of a body cavity is illuminated, to the ultrasound endoscope 12, and a monitor 20 that displays the ultrasound image and the endoscope image. The ultrasonography system 10 comprises a water supply tank 21a that stores cleaning water or the like, and a suction pump 21b that sucks aspirates inside the body cavity.

The ultrasound endoscope 12 has an insertion part 22 that is inserted into the body cavity of the subject, an operating part 24 that is consecutively provided in a proximal end portion of the insertion part 22 and is used by an operator to perform an operation, and a universal cord 26 that has one end connected to the operating part 24.

In the operating part 24, an air/water supply button 28a that opens and closes an air/water supply pipe line (not shown) from the water supply tank 21a, and a suction button 28b that opens and closes a suction pipe line (not shown) from the suction pump 21b are provided side by side. In the operating part 24, a pair of angle knobs 29 and 29 and a treatment tool insertion port 30 are provided.

In the other end portion of the universal cord 26, an ultrasound connector 32a that is connected to the ultrasound processor device 14, an endoscope connector 32b that is connected to the endoscope processor device 16, and a light source connector 32c that is connected to the light source device 18 are provided. The ultrasound endoscope 12 is attachably and detachably connected to the ultrasound processor device 14, the endoscope processor device 16, and the light source device 18 respectively through the connectors 32a, 32b, and 32c. The connector 32c comprises an air/water supply tube 34a that is connected to the water supply tank 21a, and a suction tube 34b that is connected to the suction pump 21b.

The insertion part 22 has, in order from a distal end side, a distal end part 40 that is formed of a rigid member and has an ultrasound observation part 36 and an endoscope observation part 38, a bending part 42 that is consecutively provided on a proximal end side of the distal end part 40, and a soft part 44 that connects a proximal end side of the bending part 42 and a distal end side of the operating part 24. The bending part 42 is made by connecting a plurality of bending pieces (angle rings) and is configured to be freely bent. The soft part 44 is slender and long, and has flexibility.

The bending part 42 is remotely bent and operated by rotationally moving and operating a pair of angle knobs 29 and 29 provided in the operating part 24. With this, the distal end part 40 can be directed in a desired direction.

The ultrasound processor device 14 generates and supplies an ultrasound signal for making a plurality of ultrasound oscillators 48 of an ultrasound transducer 46 (see Fig. 2) of the ultrasound observation part 36 described below generate ultrasonic waves. The ultrasound processor device 14 receives and acquires an echo signal reflected from an observation target part irradiated with the ultrasonic wave, by the ultrasound oscillator 48 and executes various kinds of signal processing on the acquired echo signal to generate an ultrasound image. The generated ultrasound image is displayed on the monitor 20.

The endoscope processor device 16 receives and acquires an image signal acquired from the observation target part illuminated with illumination light from the light source device 18 in the endoscope observation part 38 and executes various kinds of signal processing and image processing on the acquired image signal to generate an endoscope image. The generated endoscope image is displayed on the monitor 20.

The ultrasound processor device 14 and the endoscope processor device 16 are configured with two devices (computers) provided separately. Note that the present invention is not limited thereto, and both the ultrasound processor device 14 and the endoscope processor device 16 may be configured with one device.

To image an observation target part inside a body cavity using the endoscope observation part 38 to acquire an image signal, the light source device 18 generates illumination light, such as white light including light of three primary colors of red light, green light, and blue light or light of a specific wavelength. Light propagates through a light guide (not shown) and the like in the ultrasound endoscope 12, and is emitted from the endoscope observation part 38, and the observation target part inside the body cavity is illuminated with light.

The monitor 20 receives video signals generated by the ultrasound processor device 14 and the endoscope processor device 16 and displays an ultrasound image and an endoscope image. In regard to the display of the ultrasound image and the endoscope image, only one image may be appropriately switched and displayed on the monitor 20 or both images may be displayed simultaneously.

In the embodiment, although the ultrasound image and the endoscope image are displayed on one monitor 20, a monitor for ultrasound image display and a monitor for endoscope image display may be provided separately. Alternatively, the ultrasound image and the endoscope image may be displayed in a display form other than the monitor 20, for example, in a form of being displayed on a display of a terminal carried with the operator.

Next, the configuration of the distal end part 40 will be described referring to Figs. 2 and 3.

As shown in Fig. 2, the distal end part 40 of the ultrasound endoscope 12 is provided with the ultrasound observation part 36 that acquires the ultrasound image on the proximal end side, and the endoscope observation part 38 that acquires the endoscope image on the distal end side.

The distal end part 40 of the ultrasound endoscope 12 comprises a cap-shaped distal end component 41a that covers a part of the endoscope observation part 38 on the distal end side, a proximal end-side ring 41b that is disposed on the proximal end side of the ultrasound observation part 36 on the proximal end side, and a metal ring 41c (see Fig. 3), such as stainless steel (Steel Use Stainless (SUS)). Here, the distal end component 41a and the proximal end-side ring 41b are made of a rigid member, such as rigid resin, and serve as an exterior member. The metal ring 41c is disposed on an inner side of the exterior member.

The endoscope observation part 38 includes a treatment tool outlet port 76, the observation window 78, illumination windows 80, a cleaning nozzle 82, and the like provided in the distal end surface. Two illumination windows 80 are provided while sandwiching the observation window 78.

The ultrasound observation part 36 is configured with the ultrasound transducer 46. The ultrasound transducer 46 is configured by arranging a plurality of ultrasound oscillators 48 in a circumferential direction.

Inside the distal end part 40, a balloon (not shown) into which an ultrasonic wave transmission medium (for example, water or oil) covering the ultrasound observation part 36 is injected may be attachably and detachably mounted. The ultrasonic waves and the echo signals are attenuated in the air. For this reason, the balloon is expanded by injecting the ultrasonic wave transmission medium into the balloon, and is brought into contact with the observation target part, whereby it is possible to eliminate air from a region between the ultrasound transducer 46 of the ultrasound observation part 36 and the observation target part, and to prevent attenuation in the ultrasonic waves and the echo signals.

As shown in Fig. 3, in the distal end part 40, an observation system unit 85 is disposed rearward of the observation window 78 (proximal end side). The observation system unit 85 includes, for example, an objective lens 86, a prism 88, an imaging element 90, a substrate 92, and signal cables 94.

Reflected light of the observation target part incident from the observation window 78 is taken in by the objective lens 86. An optical path of the taken-in reflected light is folded at a right angle by the prism 88, and the reflected light forms an image on an imaging surface of the imaging element 90. The imaging element 90 photoelectrically converts the reflected light of the observation target part that has been transmitted through the observation window 78, the objective lens 86, and the prism 88 has formed the image on the imaging surface, to output an image signal. Examples of the imaging element 90 include a charge coupled device (CCD) and a complementary metal oxide semiconductor (CMOS).

The imaging element 90 is mounted on the substrate 92. A circuit pattern (not shown) that is electrically connected to the imaging element 90 is formed on the substrate 92. The circuit pattern comprises a plurality of electrodes in an end portion, and a plurality of signal cables 94 are connected to a plurality of electrodes. The signal cables 94 may be configured with cables in which a core wire is coated with an insulating tube. Since the circuit pattern of the substrate 92 also transmits a signal of an endoscope image, the circuit pattern of the substrate 92 and the signal cables 94 serve as a signal transmission path of the endoscope image. A plurality of signal cables 94 extend toward the bending part 42 and the soft part 44 (not shown). A plurality of signal cables 94 are inserted into the universal cord 26 from the operating part 24, and are finally connected to the endoscope connector 32b (see Fig. 1). The endoscope connector 32b is connected to the endoscope processor device 16. It is preferable that a plurality of signal cables 94 are covered with a shield member and provided as shield cables.

The treatment tool outlet port 76 is an outlet of a forceps tube 84. The forceps tube 84 extends toward a proximal end side of the insertion part 22 and extends to communicate with the treatment tool insertion port 30 of the operating part 24. A treatment tool is inserted into the forceps tube 84 from the treatment tool insertion port 30 of the operating part 24 and protrudes from the treatment tool outlet port 76. Treatment of the subject is performed by the treatment tool.

An emission end of a light guide 98 is connected to the illumination windows 80 (see Fig. 2). The light guide 98 is provided to extend from the insertion part 22 to the operating part 24. An incidence end of the light guide 98 is connected to the light source device 18 connected through the universal cord 26. The light guide 98 extends toward the proximal end side of the insertion part 22, is inserted into the universal cord 26 from the operating part 24, and is finally connected to the light source connector 32c. The light source connector 32c is connected to the light source device 18 (see Fig. 1). Illumination light emitted from the light source device 18 propagates through the light guide 98, and the observation target part is irradiated with the illumination light from the illumination windows 80.

An air/water supply channel 100 is connected to the cleaning nozzle 82. The air/water supply channel 100 extends toward the proximal end side of the insertion part 22 and is inserted into the universal cord 26 from the operating part 24. The air/water supply channel 100 is connected to the light source connector 32c and is connected to the water supply tank 21a through the air/water supply tube 34a. To clean the surfaces of the observation window 78 and the illumination windows 80, the cleaning nozzle 82 ejects air or cleaning water from the water supply tank 21a toward the observation window 78 and the illumination windows 80 through the air/water supply channel 100 in the ultrasound endoscope 12.

On the inner side of the metal ring 41c, components configuring the endoscope observation part 38, and various members, such as a pipe line extending from the proximal end side to a distal end side of the insertion part 22 and a transmission path, are stored.

The ultrasound transducer 46 configuring the ultrasound observation part 36 includes a plurality of ultrasound oscillators 48 (see Fig. 2) arranged in a cylindrical shape, an electrode part 52 including a plurality of individual electrodes 52a corresponding to a plurality of ultrasound oscillators 48 and a common electrode 52b common to a plurality of ultrasound oscillators 48, a flexible print substrate 56 to which each of a plurality of individual electrodes 52a is connected, and the metal ring 41c that supports a plurality of ultrasound oscillators 48 wound around the outer periphery thereof. The flexible print substrate 56 is also referred to as a flexible printed circuit (FPC) substrate.

The flexible print substrate 56 is thin and flexible, and thus, can be easily bent. Instead of the flexible print substrate 56, a rigid substrate that is not flexible and has high rigidity can be applied. In a case where the flexible print substrate 56 and the rigid substrate are included, simply referred to as a substrate.

The ultrasound transducer 46 further has an acoustic matching layer 64 laminated on the ultrasound oscillators 48, and an acoustic lens 66 laminated on the acoustic matching layer 64. The ultrasound transducer 46 consists of a laminate of the acoustic lens 66, the acoustic matching layer 64, the ultrasound oscillators 48, and a backing material layer 54. The laminate is coupled with the metal ring 41c by a method, such as fitting.

The acoustic matching layer 64 is provided on the outer periphery of the ultrasound oscillators 48 for taking acoustic impedance matching between the subject, such as a human body, and the ultrasound oscillators 48.

The acoustic lens 66 that is attached onto the outer periphery of the acoustic matching layer 64 is provided for converging the ultrasonic waves emitted from the ultrasound oscillator 48 toward the observation target part. The acoustic lens 66 consists of, for example, silicon-based resin (millable type silicon rubber (HTV rubber), liquid silicon rubber (RTV rubber), or the like), butadiene-based resin, or polyurethane-based resin. To take acoustic impedance matching between the subject and the ultrasound oscillator 48 by the acoustic matching layer 64, and to increase the transmittance of ultrasonic waves, powder, such as titanium oxide, alumina, or silica, is mixed in the acoustic lens 66 as needed.

As shown in Fig. 2, the ultrasound oscillators 48 are an array of a plurality of channels, for example, 48 to 192 channels (CH) consisting of a plurality of ultrasound oscillators, for example, 48 to 192 rectangular parallelepiped ultrasound oscillators 48 arranged in a cylindrical shape.

In the ultrasound transducer 46, as an example, a plurality of ultrasound oscillators 48 are arranged at predetermined pitches in a peripheral direction like the example shown in the drawing. In this way, the ultrasound oscillators 48 configuring the ultrasound transducer 46 are arranged at regular intervals in a cylindrical shape around a central axis direction (a longitudinal axis direction of the insertion part 22) of the distal end part 40. The ultrasound oscillators 48 are sequentially driven based on a drive signal input from the ultrasound processor device 14. Thus, radial electronic scanning is performed with a range in which the ultrasound oscillators 48 are arranged, as a scanning range.

As shown in Fig. 3, the flexible print substrate 56 that is attached to a side surface on the proximal end side of the backing material layer 54 is electrically connected to a plurality of individual electrodes 52a of the electrode part 52 on one side, and is connected to wiring of a plurality of coaxial cables 58 of the signal wire bundle 72 on the other side. In this manner, the individual electrodes 52a of the ultrasound oscillators 48 and the coaxial cables 58 are electrically connected, and the ultrasound oscillators 48 and the signal wire bundle 72 are electrically connected.

In the embodiment, as described below, a plurality of signal wire bundles 72 are used. The ultrasound endoscope further comprises a receiving member 110 and a pressing member 120 that are positioned on a distal end side of a plurality of signal wire bundles 72. Inside the distal end part 40, the receiving member 110 is disposed on an outer peripheral side of the distal end part 40, and the pressing member 120 is disposed on an inner side of the distal end part 40. A plurality of signal wire bundles 72 are fixed by being sandwiched between the receiving member 110 and the pressing member 120.

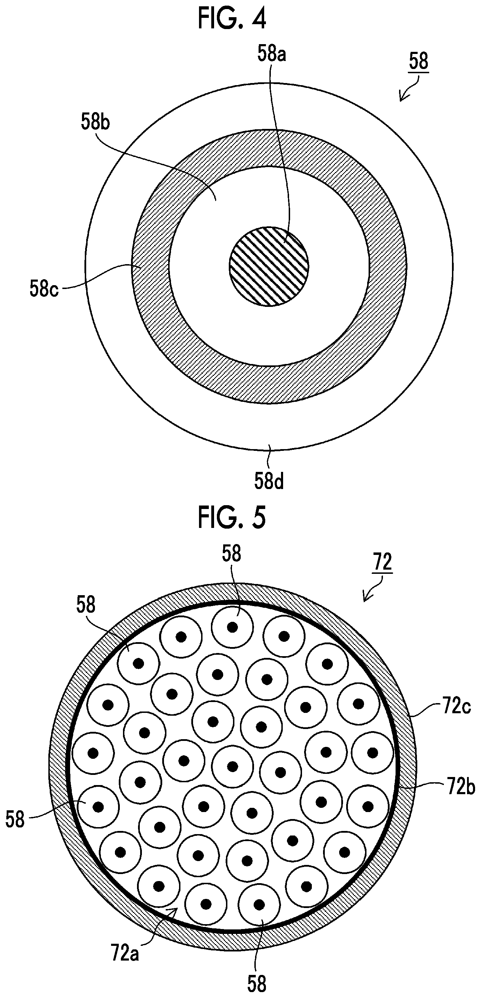

Next, the structures of the coaxial cable and the signal wire bundle will be described referring to Figs. 4 and 5.

As shown in Fig. 4, the coaxial cable 58 comprises a core wire 58a at the center, a first insulation layer 58b on the outer periphery of the core wire 58a, a shield member 58c on the outer periphery of the first insulation layer 58b, and a second insulation layer 58d on the outer periphery of the shield member 58c. The coaxial cable 58 has a structure in which the core wire 58a, the first insulation layer 58b, the shield member 58c, and the second insulation layer 58d are laminated in a concentric circular shape from the center side.

As shown in Fig. 5, the signal wire bundle 72 comprises a cable bundle 72a configured with a plurality of coaxial cables 58, a shield layer 72b with which the cable bundle 72a is coated, and an outer coat 72c with which the shield layer 72b is coated. The cable bundle 72a may be configured by stranding a plurality of coaxial cables 58. The signal wire bundle 72 is handled as one signal wire bundle 72 including a plurality of coaxial cables 58 inside.

The shield layer 72b may be configured by, for example, braiding a plurality of element wires. The element wire is made of a copper wire, a copper alloy wire, or the like subjected to plating processing (tin plating or silver plating).

A tape wound layer (not shown) may be disposed on the outer periphery of the cable bundle 72a on the inner side of the shield layer 72b. The tape wound layer is, for example, a resin tape and can suppress separation of the cable bundle 72a into the individual coaxial cables 58. In this case, a range of the tape wound layer is basically the same as a range in a longitudinal axis direction in which the cable bundle 72a is bound.

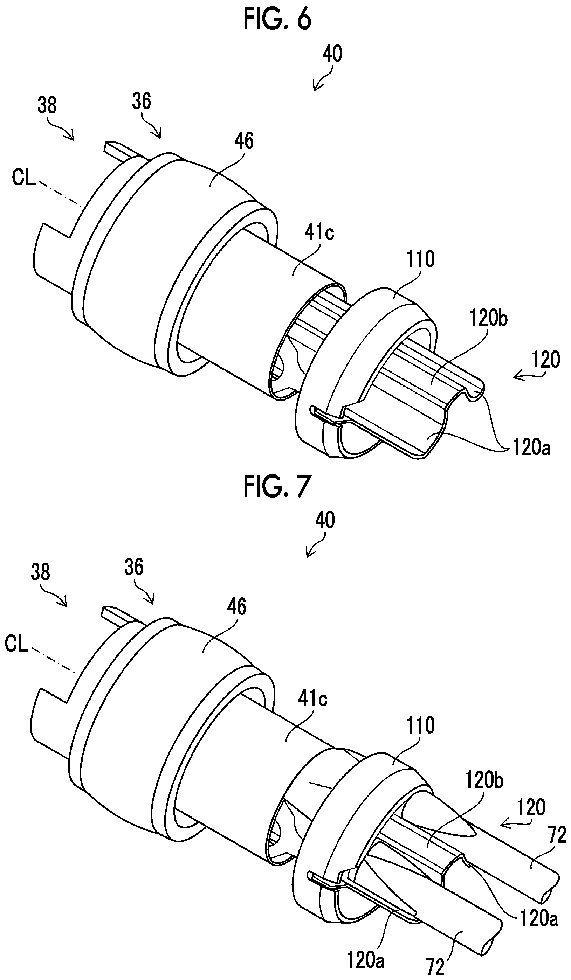

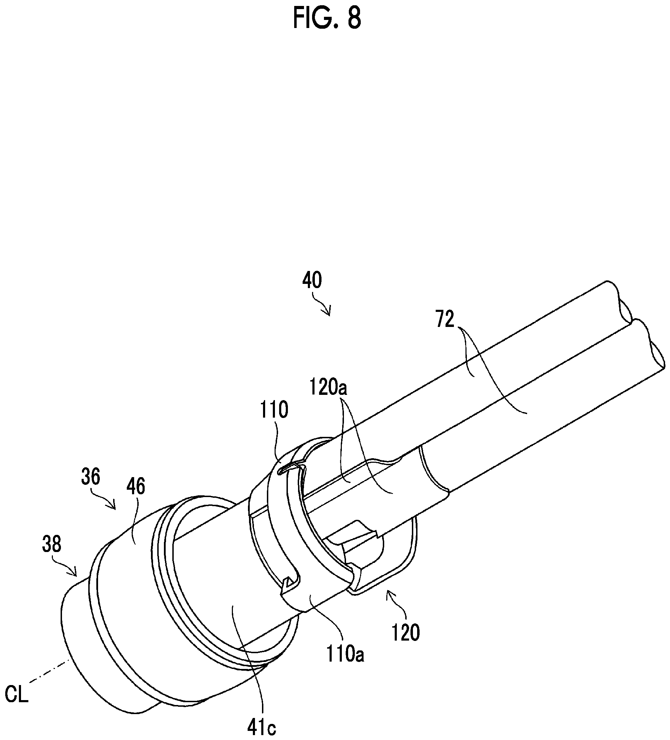

Next, the receiving member 110 and the pressing member 120 will be described referring to Figs. 6 to 8. Fig. 6 is a perspective view showing a part of the distal end part of the ultrasound endoscope. Figs. 7 and 8 are perspective views showing a part of the distal end part of the ultrasound endoscope including a plurality of signal wire bundles.

In Fig. 6, for ease of understanding of the structure of the receiving member 110 and the pressing member 120, the signal wire bundle 72 is not shown.

As shown in Fig. 6, the receiving member 110 is a member that is disposed along the inner periphery of the proximal end-side ring 41b (not shown) of the distal end part 40 and has a cylindrical shape. The cylindrical shape of the receiving member 110 includes a completed closed cylindrical shape and a cylindrical shape a part of which is cut along a central axis CL (see Fig. 8). The receiving member 110 may be disposed at least at a position facing a plurality of signal wire bundles 72.

The pressing member 120 is configured with a member extending along the central axis CL. The pressing member 120 comprises at least one a pressing surface capable of sandwiching a plurality of signal wire bundles 72 at a position facing the receiving member 110. As described below, a plurality of signal wire bundles 72 can be sandwiched by the receiving member 110 and the pressing member 120. The pressing member 120 may comprise a plurality of individual pressing surfaces 120a capable of individually pressing a plurality of signal wire bundles 72 for each signal wire bundle at least at the position facing the receiving member 110. The pressing member 120 shown in Fig. 6 comprises two individual pressing surfaces 120a capable of individually pressing two signal wire bundles 72 for each signal wire bundle. It is preferable that the individual pressing surface 120a has a curved shape following each signal wire bundle 72 of a plurality of signal wire bundles 72. The individual pressing surface 120a in the curved shape can reliably sandwich and fix each signal wire bundle 72 in cooperation with the receiving member 110.

The individual pressing surface 120a may extend to the proximal end side beyond the receiving member 110. An area where the signal wire bundle 72 is in contact with the individual pressing surface 120a increases, and the signal wire bundle 72 can be stably sandwiched.

The pressing member 120 comprises a connecting portion 120b that connects the two individual pressing surfaces 120a arranged along the central axis CL. The connecting portion 120b connects end portions in a width direction of the two individual pressing surfaces 120a. With this, a space where the signal wire bundle 72 extends along the central axis CL is defined between the two individual pressing surfaces 120a on an opposite side from the signal wire bundle 72 with the pressing member 120 interposed therebetween. The connecting portion 120b may extend to the distal end side beyond the receiving member 110. In the embodiment, the connecting portion 120b extends to the position of the metal ring 41c. In the pressing member 120, the two individual pressing surfaces 120a and the connecting portion 120b can be integrally manufactured.

As shown in Figs. 7 and 8, the two signal wire bundles 72 are sandwiched and fixed by the receiving member 110 and the pressing member 120. As shown in Figs. 6 to 8, a plurality of signal wire bundles 72 can be fixed by the receiving member 110 and the pressing member 120 having comparatively simple structures.

In general, in an ultrasound endoscope, 100 or more coaxial cables are bundled, are coated with an outer coat, and are electrically connected as one signal wire bundle to an ultrasound transducer. It is desirable that such a signal wire bundle is locally deformed in shape (in an elliptical shape in cross sectional view) at a position of the distal end part 40 of the ultrasound endoscope 12 to eliminate a dead space. However, since the outer coat of the signal wire bundle is rigid, even though the signal wire bundle is deformed in an elliptical shape using a shrinkable tube, the signal wire bundle may be returned to an original circular shape. Accordingly, it is not easy to maintain a state in which the signal wire bundle is deformed. In a case where the deformed shape of the signal wire bundle cannot be maintained, since the signal wire bundle is thick among the contents disposed in the distal end part, the dead space increases.

Accordingly, in the embodiment, one signal wire bundle is formed of a plurality of signal wire bundles 72, whereby force needed for deforming the signal wire bundles 72 is made small. A plurality of signal wire bundles 72 are sandwiched and fixed between the receiving member 110 disposed on the outer peripheral side of the distal end part 40 and the pressing member 120 disposed on the inner side, whereby it is possible to easily deform a plurality of signal wire bundles 72 and to maintain the shape of the deformed signal wire bundles 72.

At least one of the receiving member 110 or the pressing member 120 is made of metal, and thus, can be reduced in thickness. Examples of a material of metal include SUS and aluminum. It is preferable that both the receiving member 110 and the pressing member 120 are made of metal.

The receiving member 110 and the pressing member 120 are made of a rigid body, whereby it is possible to more reliably maintain and fix the deformation of the signal wire bundles 72. The rigid body may have at least rigidity enough to deform the signal wire bundles 72 or may have higher rigidity.

A plurality of signal wire bundles 72 are formed and the force needed for deforming the signal wire bundles 72 is made small, whereby it is possible to deform the signal wire bundles 72 even though the receiving member 110 and the pressing member 120 is reduced in thickness, and as a result, it is possible to reduce the diameter of the distal end part 40 of the ultrasound endoscope 12.

As shown in Fig. 8, the receiving member 110 may have the connecting portion 110a extending to the distal end side. It is possible to connect the receiving member 110 and the metal ring 41c through the connecting portion 1 10a. A relative positional relationship of the receiving member 110 and the metal ring 41c is decided.

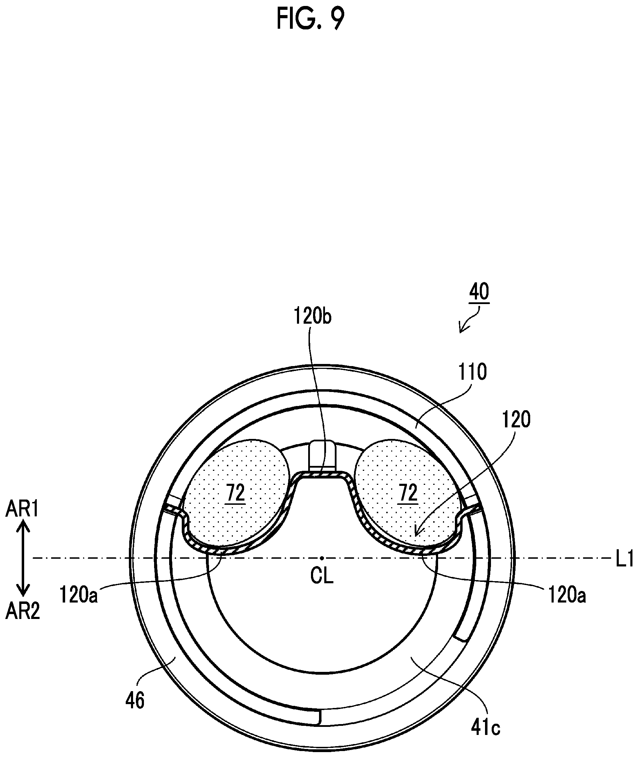

Next, preferred disposition of the signal wire bundles 72, the forceps tube 84, and the signal cable 94 at a position in the distal end part 40 where the receiving member 110 and the pressing member 120 are disposed will be described referring to Figs. 9 to 12.

First, the disposition of the signal wire bundle 72 will be described. Fig. 9 shows a distal end part cross section taken along a plane that is perpendicular to the central axis CL of the distal end part 40 and passes through the receiving member 110 and the pressing member 120. A plurality of signal wire bundles 72 are disposed in a direction extending along the central axis CL of the distal end part 40. The distal end part cross section is divided into two areas by a straight line L1 passing through the central axis CL. In a case where one area of the two divided areas is defined as a first divided area AR1, and the other area is defined as a second divided area AR2, a plurality of signal wire bundles 72 are disposed in the first divided area AR1 as one area.

As shown in Fig. 9, a plurality of signal wire bundles 72 are deformed in an elliptical shape in cross sectional view by the receiving member 110 and the pressing member 120. As a result, it is possible to reduce a dead space that occurs between the inner periphery of the receiving member 110 and the outer periphery of the signal wire bundle 72.

Since a plurality of signal wire bundles 72 are disposed in the first divided area AR1, it is possible to increase a disposition space in inserting the contents other than the signal wire bundle 72 in the distal end part 40. It is possible to reduce the dead space of the distal end part 40 by effectively utilizing the disposition space, and to efficiently dispose the contents in the distal end part 40, and as a result, it is possible to reduce the diameter of the distal end part 40.

The curved shape of the individual pressing surface 120a corresponding to each signal wire bundle 72 does not need to completely follow the signal wire bundle 72, and may have a curved shape enough to deform the signal wire bundle 72 in a substantially elliptical shape.

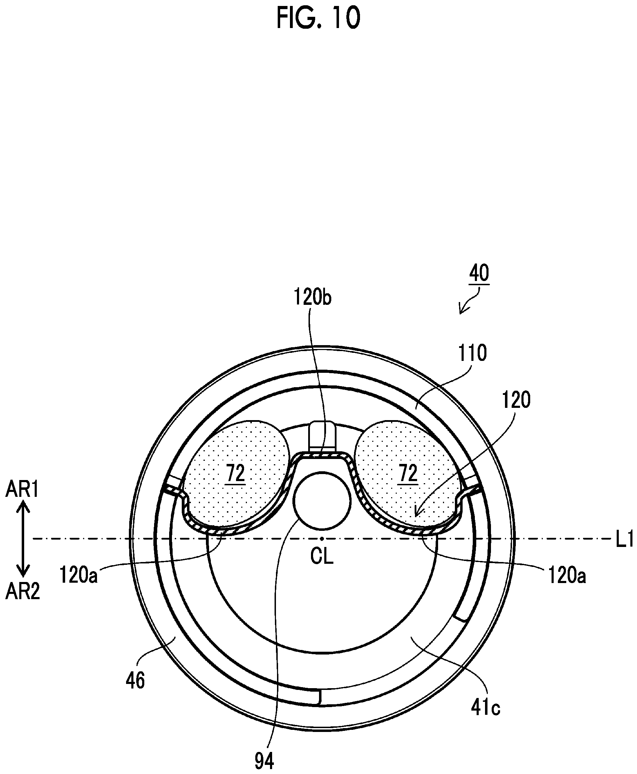

Next, the disposition of the signal wire bundles 72 and the signal cable 94 will be described. Fig. 10 shows a distal end part cross section similar to Fig. 9. As shown in Fig. 10, in the distal end part 40, a plurality of signal wire bundles 72 and the signal cable 94 are disposed along the central axis CL. The signal cable 94 is disposed between the two signal wire bundles 72. The two signal wire bundles 72 are sandwiched and fixed by the receiving member 110 and the pressing member 120. The signal cable 94 is disposed in a space defined by disposing the two signal wire bundles 72 in parallel, whereby it is possible to effectively utilize the disposition space of the distal end part 40, and to reduce the diameter of the distal end part 40.

It is possible to realize improvement of the operability of an operator. That is, in the embodiment, it is possible to dispose an optical system, such as the objective lens 86, in the observation system unit 85 near the central axis CL of the distal end part 40, and to reduce a sense of discomfort when the operator operates the ultrasound endoscope 12. The reason is that, as the optical system is closer to an end portion of the outer periphery of the distal end part 40, in a case of entering the ultrasound endoscope 12 inside a lumen, the operator has a feeling that the ultrasound endoscope 12 seems to enter the corner of the lumen.

As shown in Fig. 10, it is preferable that, in the distal end part cross section, a plurality of signal wire bundles 72 and the signal cable 94 are disposed in the first divided area AR1. Since the signal wire bundles 72 and the signal cable 94 are disposed in the first divided area AR1, it is possible to more effectively utilize the disposition space of the distal end part 40.

The signal cable 94 is disposed between a plurality of individual pressing surfaces 120a on an opposite side from a plurality of signal wire bundles 72 with the pressing member 120 interposed therebetween. A plurality of signal wire bundles 72 are reliably sandwiched by the receiving member 110 and the pressing member 120. Since the signal cable 94 is not sandwiched by the receiving member 110 and the pressing member 120, it is possible to provide the signal cable 94 with a certain degree of freedom for disposition.

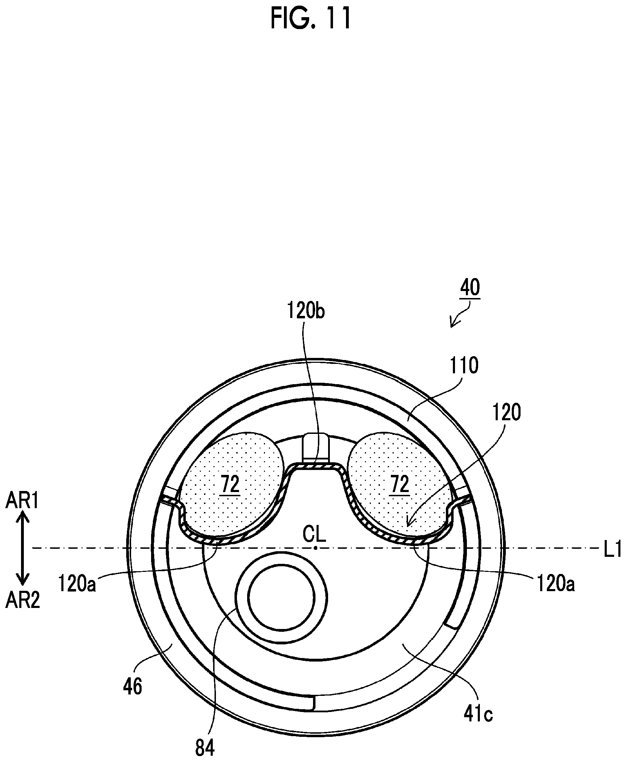

Next, the disposition of the signal wire bundles 72 and the forceps tube 84 will be described. Fig. 11 shows a distal end part cross section similar to Fig. 9. As shown in Fig. 11, in the distal end part 40, a plurality of signal wire bundles 72 and the forceps tube 84 are disposed along the central axis CL. The two signal wire bundles 72 are sandwiched and fixed by the receiving member 110 and the pressing member 120. In the distal end part cross section, a plurality of signal wire bundles 72 are disposed in the first divided area AR1, and the forceps tube 84 is disposed in the second divided area AR2. The forceps tube 84 is a content having the greatest thickness next to the signal wire bundles 72, and the forceps tube 84 and the signal wire bundles 72 are disposed in the first divided area AR1 and the second divided area AR2 without interfering with each other, respectively, whereby it is possible to more effectively utilize the disposition space of the distal end part 40, and to reduce the diameter of the distal end part 40.

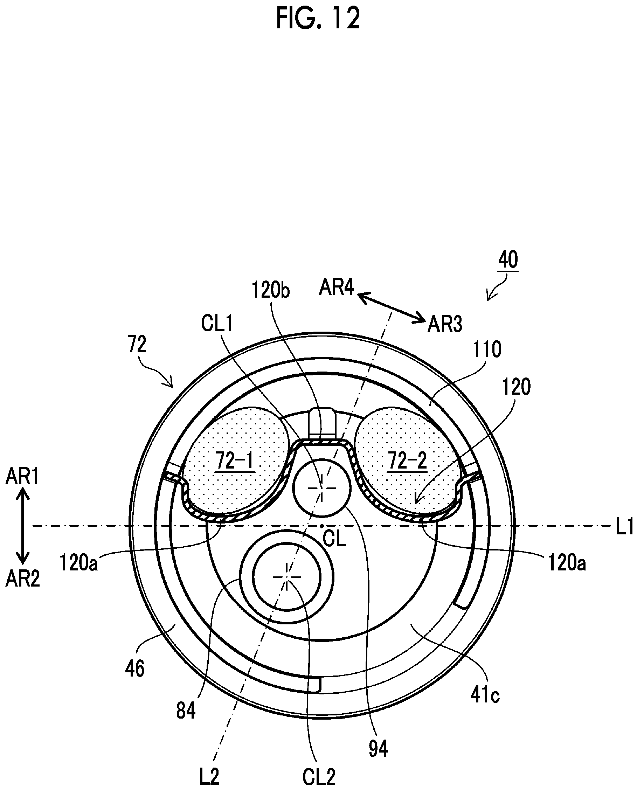

Next, the disposition of the signal wire bundles 72, the signal cable 94, and the forceps tube 84 will be described. Fig. 12 shows a distal end part cross section similar to Fig. 9. As shown in Fig. 12, in the distal end part 40, a plurality of signal wire bundles 72, the signal cable 94, and the forceps tube 84 are disposed along the central axis CL. The two signal wire bundles 72 (72-1 and 72-2) are sandwiched and fixed by the receiving member 110 and the pressing member 120.

As shown in Fig. 12, in the distal end part cross section, in a case where one area of areas obtained by dividing the distal end part cross section into two with a straight line L2 passing through a central axis CL1 of the signal cable 94 and a central axis CL2 of the forceps tube 84 is defined as a third divided area AR3, and the other area is defined as a fourth divided area AR4, a part of signal wire bundles 72-1 among a plurality of signal wire bundles 72 is disposed in the third divided area AR3, and the other part of signal wire bundles 72-2 is disposed in the fourth divided area AR4.

In the disposition space of the distal end part 40, the signal wire bundles 72-1 and 72-2, the signal cable 94, and the forceps tube 84 have a greater occupying range that other contents. Accordingly, the two signal wire bundles 72-1 and 72-2 are disposed in different divided areas, whereby it is possible to effectively utilize the disposition space.

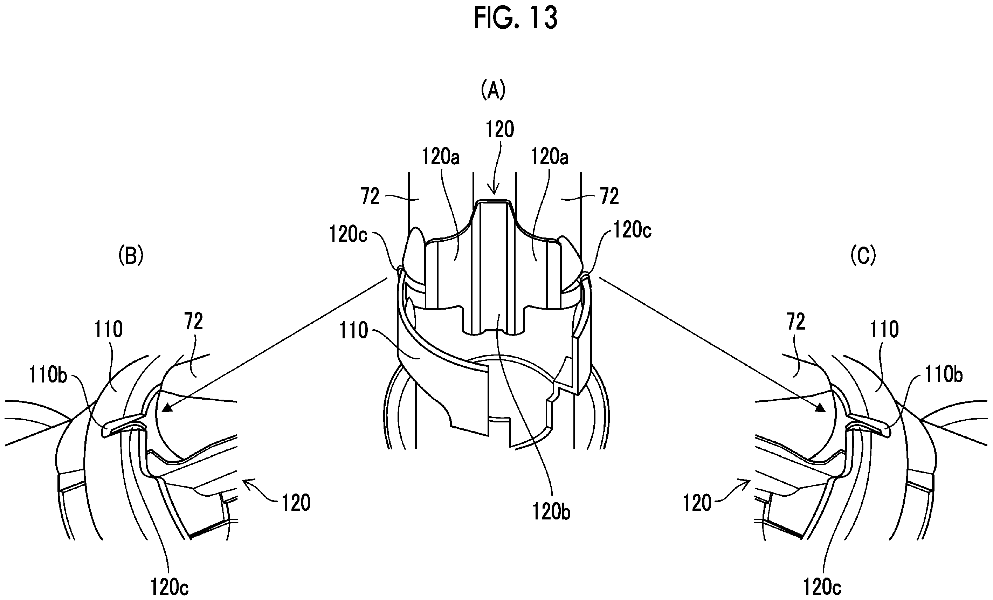

Next, a positioning structure of positioning relative positions of the receiving member 110 and the pressing member 120 will be described referring to Fig. 13.

(A) of Fig. 13 is a perspective view when a part of the distal end part 40 of the ultrasound endoscope 12 including a plurality of signal wire bundles 72 is viewed from the proximal end side. (B) of Fig. 13 and (C) of Fig. 13 are enlarged views of the positioning structure.

As shown in (A) of Fig. 13, the pressing member 120 is disposed at a position capable of sandwiching a plurality of signal wire bundles 72, and the pressing member 120 is fixed to the receiving member 110. Specifically, as shown in (B) of Fig. 13 and (C) of Fig. 13, locking grooves 110b are formed in the receiving member 110. The locking grooves 110b are slits that extend along the central axis CL and have one end open toward the proximal end side. On the other hand, the pressing member 120 is provided with locking pieces 120c lockable into the locking grooves 110b. For example, the locking pieces 120c extend in a direction away from the central axis CL continuously to the individual pressing surfaces 120a.

The locking pieces 120c are locked into the locking grooves 110b by inserting the locking pieces 120c of the pressing member 120 into the locking grooves 110b of the receiving member 110. As a result, the relative positions of the receiving member 110 and the pressing member 120 are positioned.

The positioning structure shown in Fig. 13 can eliminate a need for using an adhesive and can save a step of applying an adhesive. Note that the positioning structure permits the use of the adhesive. The positioning structure can position the receiving member 110 and the pressing member 120 and can eliminate a need for using screws with a comparatively simple structure in which the locking pieces 120c are inserted into the locking grooves 110b. As a result, it is possible to reduce the diameter of the distal end part 40. This is because, in general, in a case where screws are used, a disposition space for the screws is needed, and the diameter of the distal end part 40 is made large.

In the embodiment, a case where the locking grooves 110b are provided in the receiving member 110 and the locking pieces 120c are provided in the pressing member 120 has been described. The present invention is not limited thereto, and the locking pieces may be provided in the receiving member 110 and the locking grooves may be provided in the pressing member 120. The locking pieces of the receiving member 110 are inserted into the locking grooves of the pressing member 120, whereby it is possible to position the relative positions of the receiving member 110 and the pressing member 120.

It is preferable that the receiving member 110 and the pressing member 120 are insulated from the ground of the distal end part 40. It is preferable that the receiving member 110 and the pressing member 120 are insulated from the ground of the signal cable 94. It is possible to suppress the occurrence of noise due to a signal transmitted through the signal cable 94 of the ultrasound endoscope 12 entering the signal wire bundles 72 by insulating the above-described grounds from the receiving member 110 and the pressing member 120.

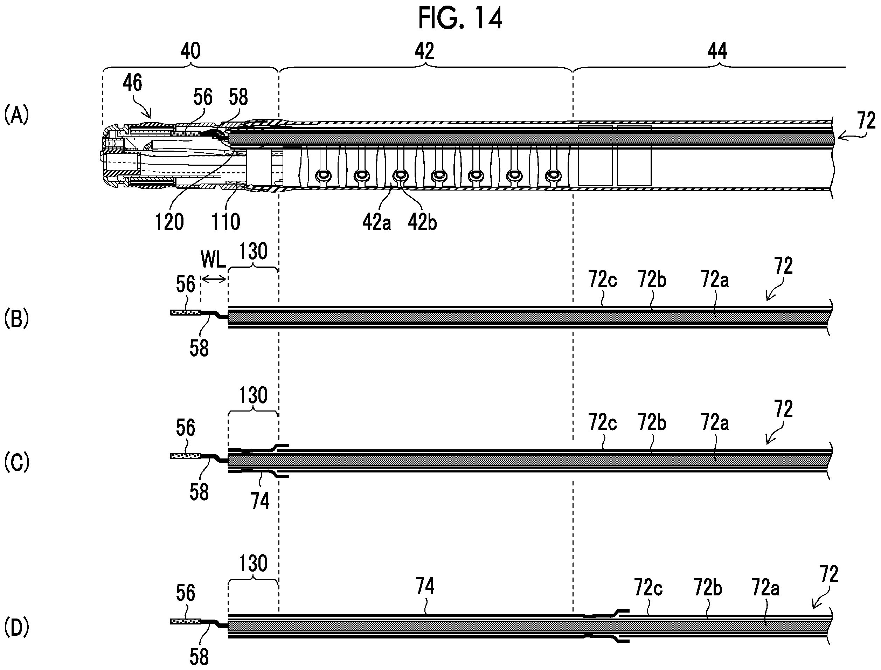

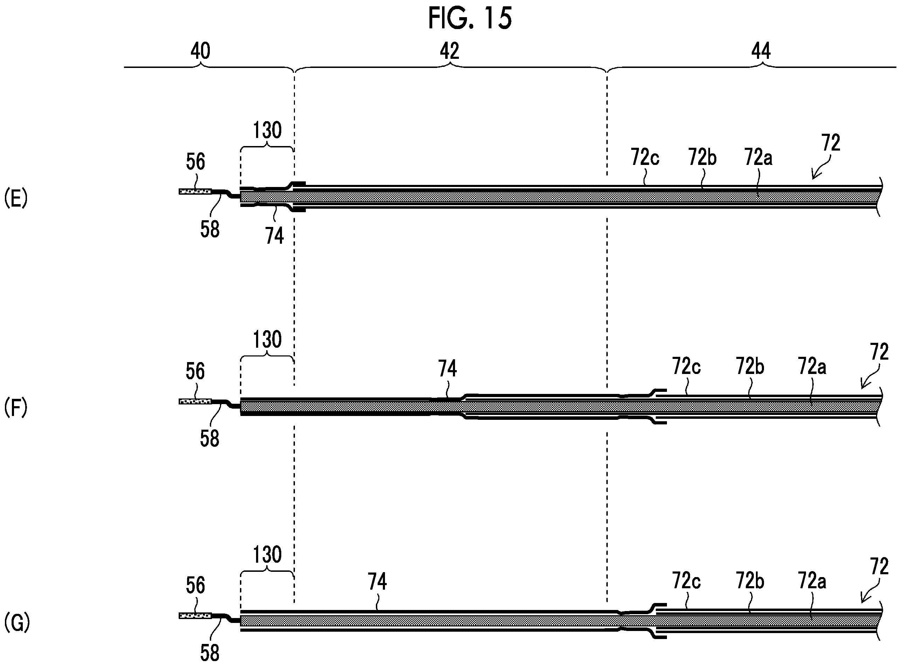

Next, a preferred structure of the signal wire bundle 72 will be described referring to Figs. 14 and 15. Fig. 14 is a schematic view showing the structures of the distal end part 40, the bending part 42, the soft part 44, and the signal wire bundle 72. (A) of Fig. 14 is a diagram showing a state in which the signal wire bundle 72 is inserted into the distal end part 40, the bending part 42, and the soft part 44. (B) of Fig. 14 shows a structure of a first form of the signal wire bundle 72, (C) of Fig. 14 shows a structure of a second form of the signal wire bundle 72, and (D) of Fig. 14 shows a structure of a third form of the signal wire bundle 72.

Fig. 15 is a schematic view showing the structure of the signal wire bundle 72. (E) of Fig. 15 shows a structure of a fourth form of the signal wire bundle 72, (F) of Fig. 15 shows a structure of a fifth form of the signal wire bundle 72, and (G) of Fig. 15 shows a structure of a sixth form of the signal wire bundle 72.

As shown in (A) of Fig. 14, the signal wire bundle 72 is inserted into the soft part 44, the bending part 42, and the distal end part 40. In the distal end part 40, the coaxial cables 58 are electrically connected to the flexible print substrate 56. The signal wire bundle 72 is sandwiched by the receiving member 110 and the pressing member 120 (hereinafter, a portion sandwiched by the receiving member 110 and the pressing member 120 is referred to as a sandwiching portion 130). A plurality of bending pieces 42a are connected to the bending part 42 through connecting portions 42b in an axial direction. Inside a plurality of bending pieces 42a, a plurality of operation wires (not shown) are disposed along an axial direction and are disposed at predetermined intervals in a peripheral direction. A proximal end of the operation wire is connected to a pulley (not shown) that is rotationally moved by a pair of angle knobs 29 and 29 provided in the operating part 24. With this, in a case where a pair of angle knobs 29 and 29 is operated to be rotationally moved to rotationally move the pulley, the operation wires are pulled, and the bending part 42 is bent in a desired direction.

As shown in (B) of Fig. 14, the first form of the signal wire bundle 72 comprises a cable bundle 72a configured with a plurality of coaxial cables 58, a shield layer 72b with which the cable bundle 72a is coated, and an outer coat 72c with which the shield layer 72b is coated. In the signal wire bundle 72, the shield layer 72b and the outer coat 72c for a given length from the sandwiching portion 130 to the distal end side are removed. The cable bundle 72a is exposed from the outer coat 72c and the shield layer 72b. A plurality of coaxial cables 58 configuring the cable bundle 72a are loosened into the individual coaxial cables 58, and each coaxial cable 58 is electrically connected to an electrode pad (not shown) of the flexible print substrate 56.

As shown in (B) of Fig. 14, in the distal end part 40, since the outer coat 72c and the shield layer 72b for at least a given length from the sandwiching portion 130 are removed, it is possible to secure a wiring length WL (a length from the end portion of the outer coat 72c to the distal end of the coaxial cable 58) at the time of wiring work. With this, it becomes easy to handle the coaxial cable 58 at the time of wiring, and it is possible to reduce wiring miss, such as disconnection. Distal end positions of the shield layer 72b and the outer coat 72c are in the distal end part 40, and both the distal end positions coincide with a distal end position of the cable bundle 72a.

As shown in (C) of Fig. 14, the second form of the signal wire bundle 72 is different from the first form of the signal wire bundle 72 in that the outer coat 72c is removed in the distal end part 40. The shield layer 72b is exposed in an exposed area exposed from the outer coat 72c. In the exposed area (shield layer 72b), an insulating member 74 that has rigidity lower than the outer coat 72c and with which the exposed area (shield layer 72b) is coated is provided. Examples of the insulating member 74 include a silicon tube, a polytetrafluoroethylene (PTFE) tube, a heat-shrinkable tube, and a tape. Since the outer coat 72c of the signal wire bundle 72 is rigid, the outer coat 72c is removed and the exposed area is coated with the insulating member 74 having rigidity lower than the outer coat 72c, whereby it is possible to easily deform the signal wire bundle 72, and to maintain the deformed shape in the sandwiching portion 130. The distal end position of the outer coat 72c is in the bending part 42 and does not coincide with the distal end position of the shield layer 72b. The distal end position of the shield layer 72b coincides with the distal end position of the cable bundle 72a.

As shown in (D) of Fig. 14, the third form of the signal wire bundle 72 is different from the first form of the signal wire bundle 72 in that the outer coat 72c is removed in a part of the distal end part 40, the bending part 42, and the soft part 44. Similarly to the second form, the shield layer 72b is exposed in the exposed area exposed from the outer coat 72c. In the exposed area (shield layer 72b), an insulating member 74 that has rigidity lower than the outer coat 72c and with which the exposed area (shield layer 72b) is coated is provided.

In the third form of the signal wire bundle 72, similarly to the second form, since the outer coat 72c of the sandwiching portion 130 is removed, it is possible to easily deform the signal wire bundle 72, and to maintain the deformed shape. In the third form of the signal wire bundle 72, since the rigid outer coat 72c is removed in the bending part 42, it is possible to easily bend the bending part 42. The distal end position of the outer coat 72c is in the soft part 44 and does not coincide with the distal end position of the shield layer 72b. The distal end position of the shield layer 72b coincides with the distal end position of the cable bundle 72a.

As shown in (E) of Fig. 15, the fourth form of the signal wire bundle 72 is different from the first form of the signal wire bundle 72 in that the shield layer 72b and the outer coat 72c are removed in the distal end part 40. Since the shield layer 72b is also removed in the exposed area exposed from the outer coat 72c, the cable bundle 72a is exposed. In the exposed area (cable bundle 72a) exposed from the outer coat 72c, an insulating member 74 that has rigidity lower than the outer coat 72c and with which the exposed area (cable bundle 72a) is coated is provided.

In the fourth form of the signal wire bundle 72, since the shield layer 72b and the outer coat 72c are removed in the sandwiching portion 130, more reliably, it is possible to easily deform the signal wire bundle 72, and to maintain the deformed shape. The distal end positions of the shield layer 72b and the outer coat 72c are in the bending part 42, and both the distal end positions coincide with each other. The distal end positions of the shield layer 72b and the outer coat 72c do not coincide with the distal end position of the cable bundle 72a.

As shown in (F) of Fig. 15, the fifth form of the signal wire bundle 72 is different from the first form of the signal wire bundle 72 in that the shield layer 72b and the outer coat 72c are removed in the distal end part 40. The shield layer 72b is removed in a part of the bending part 42. The outer coat 72c is removed in a part of the bending part 42 and the soft part 44.

In an exposed area (cable bundle 72a and shield layer 72b) exposed from the outer coat 72c, an insulating member 74 that has rigidity lower than the outer coat 72c and with which the exposed area (cable bundle 72a and shield layer 72b) are coated is provided.

In the fifth form of the signal wire bundle 72, since the shield layer 72b and the outer coat 72c are removed in the sandwiching portion 130, more reliably, it is possible to easily deform the signal wire bundle 72, and to maintain the deformed shape. In the fifth form of the signal wire bundle 72, since the rigid outer coat 72c is removed in the bending part 42, it is possible to easily bend the bending part 42. The distal end position of the shield layer 72b is in the bending part 42, and the distal end position of the outer coat 72c is in the soft part 44. Both the distal end positions do not coincide with each other.

As shown in (G) of Fig. 15, the sixth form of the signal wire bundle 72 is different from the first form of the signal wire bundle 72 in that the outer coat 72c is removed in a part of the distal end part 40, the bending part 42, and the soft part 44. The shield layer 72b is removed in a part of the distal end part 40, the bending part 42, and the soft part 44.

In the exposed area (cable bundle 72a) exposed from the outer coat 72c, an insulating member 74 that has rigidity lower than the outer coat 72c and with which the exposed area (cable bundle 72a) is coated is provided.

In the sixth form of the signal wire bundle 72, since the shield layer 72b and the outer coat 72c are removed in the sandwiching portion 130, more reliably, it is possible to easily deform the signal wire bundle 72, and to maintain the deformed shape. In the sixth form of the signal wire bundle 72, since the rigid outer coat 72c is removed in the bending part 42, it is possible to easily bend the bending part 42. The distal end positions of the shield layer 72b and the outer coat 72c are in the soft part 44, and both the distal end positions coincide with each other. The structure of the signal wire bundle 72 is not limited to the structures of Figs. 14 and 15, and can be suitably changed, and the distal end positions of the shield layer 72b and the outer coat 72c may coincide with each other or may not coincide with each other.

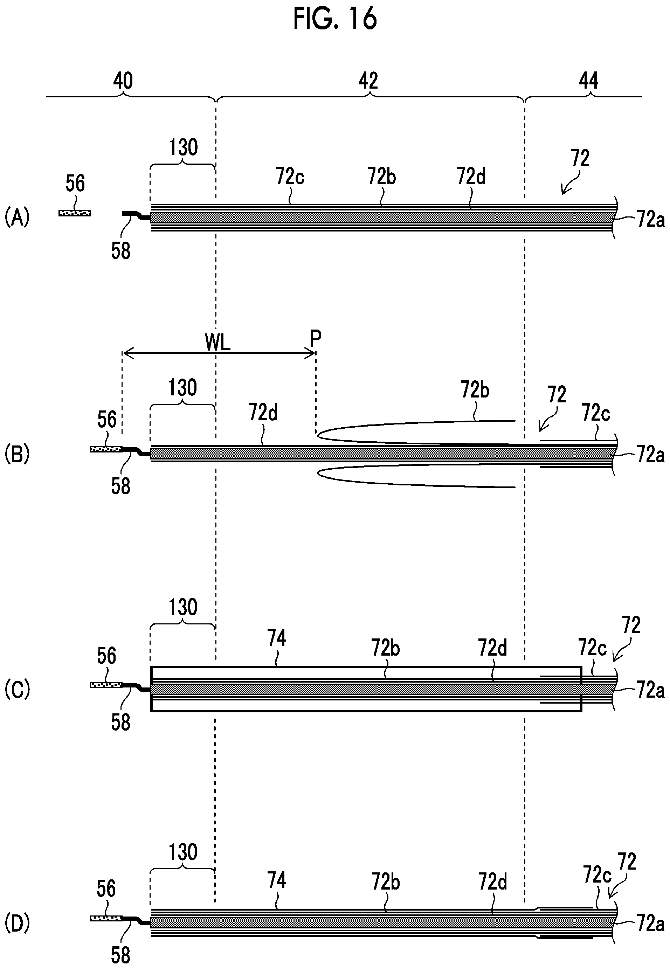

Next, a preferred procedure for electrically connecting the coaxial cables 58 of the signal wire bundle 72 to the flexible print substrate 56 will be described referring to Fig. 16.

As shown in (A) of Fig. 16, the signal wire bundle 72 and the flexible print substrate 56 are prepared. The signal wire bundle 72 comprises the cable bundle 72a configured with a plurality of coaxial cables 58, a tape 72d that bundles the cable bundle 72a, the shield layer 72b with which the cable bundle 72a bundled with the tape 72d is coated, and the outer coat 72c with which the shield layer 72b is coated. The outer coat 72c, the shield layer 72b, and the tape 72d for a given length from the sandwiching portion 130 toward the distal end side are removed. A plurality of coaxial cables 58 of the exposed cable bundle 72a are loosened into the individual coaxial cables 58.

The flexible print substrate 56 is provided with electrode pads (not shown) that are electrically connected to the coaxial cables 58 on the proximal end side. The distal end side of the coaxial cable 58 and the electrode pad of the flexible print substrate 56 are disposed at facing positions.

Next, as shown in (B) of Fig. 16, the outer coat 72c is removed in a part of the distal end part 40, the bending part 42, and the soft part 44. The shield layer 72b of the exposed area is folded back at a position P in the bending part 42 from the distal end side. In a case where the shield layer 72b is configured by, for example, braiding, since it is possible to easily fold back the shield layer 72b, there is no need to remove the shield layer 72b at the same position as the end portion of the outer coat 72c. The distal end position of the outer coat 72c is in the soft part 44.

Since the outer coat 72c and the shield layer 72b are not present between the distal end of the coaxial cable 58 and the position P, it is possible to secure a wiring length WL (a length from the position P to the distal end of the coaxial cable 58) at the time of wiring work, and to easily handle the coaxial cables 58. As a result, it is possible to reduce wiring miss, such as disconnection, in electrically connecting the coaxial cables 58 to the flexible print substrate 56.

Next, as shown in (C) of Fig. 16, the folded-back shield layer 72b is returned to a state before folding-back. The shield layer 72b is folded back to extend the wiring length WL at the time of wiring work and is returned to the original state after wiring, and the cable bundle 72a is coated with the shield layer 72b up to the distal end position thereof. With this, it is possible to improve electromagnetic compatibility (EMC) characteristics. The distal end positions of the cable bundle 72a and the shield layer 72b coincide with each other.

Thereafter, a region from a portion on the distal end side of the outer coat 72c to the distal end position of the shield layer 72b is coated with a heat-shrinkable tube as the insulating member 74. The heat-shrinkable tube is a tubular member with openings at both ends, and shrinks through heating in a direction in which the diameter thereof is made small.

Next, as shown in (D) of Fig. 16, the insulating member 74 is heated to make the insulating member 74 shrink. With this, in the exposed area (shield layer 72b) exposed from the outer coat 72c, the insulating member 74 that has rigidity lower than the outer coat 72c and with which the exposed area (cable bundle 72a and shield layer 72b) are coated is provided. It is possible to secure the length of the wiring length WL and to improve the EMC characteristics without removing the shield layer 72b.

Although a radial electronic scanning type ultrasound endoscope 12 has been described, the present invention can also be applied to a convex electronic scanning type ultrasound endoscope.

### Explanation of References

10: ultrasonography system
12: ultrasound endoscope
14: ultrasound processor device
16: endoscope processor device
18: light source device
20: monitor
21a: water supply tank
21b: suction pump
22: insertion part
24: operating part
26: universal cord
28a: air/water supply button
28b: suction button
29: angle knob
30: treatment tool insertion port
32a: connector
32b: connector
32c: connector
34a: air/water supply tube
34b: suction tube
36: ultrasound observation part
38: endoscope observation part
40: distal end part
41a: distal end component
41b: proximal end-side ring
41c: metal ring
42: bending part
42a: bending piece
42b: connecting portion
44: soft part
46: ultrasound transducer
48: ultrasound oscillator
52: electrode part
52a: individual electrode
52b: common electrode
54: backing material layer
56: flexible print substrate
58: coaxial cable
58a: core wire
58b: first insulation layer
58c: shield member
58d: second insulation layer
64: acoustic matching layer
66: acoustic lens
72: signal wire bundle
72-1: signal wire bundle
72-2: signal wire bundle
72a: cable bundle
72b: shield layer
72c: outer coat
72d: tape
74: insulating member
76: treatment tool outlet port
78: observation window
80: illumination window
82: cleaning nozzle
84: forceps tube
85: observation system unit
86: objective lens
88: prism
90: imaging element
92: substrate
94: signal cable
98: light guide
100: air/water supply channel
110: receiving member
110a: connecting portion
110b: locking groove
120: pressing member
120a: individual pressing surface
120b: connecting portion
120c: locking piece
130: sandwiching portion
AR1: first divided area
AR2: second divided area
AR3: third divided area
AR4: fourth divided area
CL: central axis
CL1: central axis
CL2: central axis
L 1: straight line
L2: straight line
P: position
WL: wiring length

## Claims

1. An ultrasound endoscope in which an ultrasound transducer (46) is disposed in a distal end part (40) of an insertion part (22), the ultrasound endoscope (12) comprising:
a plurality of signal wire bundles (72) that are electrically connected to the ultrasound transducer and extend along a central axis of the distal end part, each signal wire bundle including a plurality of coaxial cables (72a), a shield layer (72b) with which the plurality of coaxial cables are coated, and an outer coat (72c) with which the shield layer is coated;
a receiving member (110) that is positioned on a distal end side of the plurality of signal wire bundles, the receiving member being disposed on an outer peripheral side in the distal end part along an inner periphery of the distal end part, and **characterized by**:
a pressing member that is positioned on the distal end side of the plurality of signal wire bundles, the pressing member being disposed on an inner side in the distal end part and comprises at least one pressing surface, wherein the at least one pressing surface is disposed closer to the central axis than the receiving member in the distal end part,
wherein the plurality of signal wire bundles are fixed by being sandwiched between the receiving member and the pressing member.

2. The ultrasound endoscope according to claim 1,
wherein, in a distal end part cross section taken along a plane that is perpendicular to the central axis of the distal end part (40) and passes through the receiving member (110) and the pressing member (120), in a case where one area of areas obtained by dividing the distal end part cross section into two with a straight line passing through the central axis is defined as a first divided area (AR1), and the other area is defined as a second divided area (AR2), the plurality of signal wire bundles are disposed in the first divided area.

3. The ultrasound endoscope according to claim 2,
wherein an observation system unit (85) is disposed in the distal end part, and includes an imaging element (90) and a signal cable (94) that is connected to the imaging element, and
the signal cable is disposed along at least one of the plurality of signal wire bundles.

4. The ultrasound endoscope according to claim 3,
wherein, in the distal end part cross section, the plurality of signal wire bundles and the signal cable are disposed in the first divided area.

5. The ultrasound endoscope according to claim 4,
wherein the pressing member includes a plurality of individual pressing surfaces (120a) that individually press the plurality of signal wire bundles for each signal wire bundle, and each individual pressing surface has a curved shape following each signal wire bundle.

6. The ultrasound endoscope according to claim 5,
wherein the signal cable is disposed between the plurality of individual pressing surfaces on an opposite side from the plurality of signal wire bundles with the pressing member interposed therebetween.

7. The ultrasound endoscope according to any one of claims 2 to 6, further comprising:
a forceps tube (84) that is disposed in the distal end part along the central axis,
wherein, in the distal end part cross section, the plurality of signal wire bundles are disposed in the first divided area, and the forceps tube is disposed in the second divided area.

8. The ultrasound endoscope according to claim 7 when dependent on any of claims 3, 4 or 6,
wherein an observation system unit (85) is disposed in the distal end part, and includes an imaging element and a signal cable that is connected to the imaging element, and
in the distal end part cross section, in a case where one area of areas obtained by dividing the distal end part cross section into two with a straight line passing through a central axis of each of the signal cable and the forceps tube is defined as a third divided area (AR3), and the other area is defined as a fourth divided area (AR4), a part of signal wire bundles among the plurality of signal wire bundles is disposed in the third divided area, and the other part of signal wire bundles is disposed in the fourth divided area.

9. The ultrasound endoscope according to any one of claims 1 to 8,
wherein, at a position to be fixed by the receiving member and the pressing member, the plurality of signal wire bundles include an exposed area that is exposed from the outer coat, and an insulating member that has rigidity lower than the outer coat and with which the exposed area is coated.

10. The ultrasound endoscope according to any one of claims 1 to 9,
wherein a locking groove (110b) with one end open is provided in one of the receiving member and the pressing member, a locking piece (120c) lockable into the locking groove is provided in the other member of the receiving member and the pressing member, and the locking piece is locked into the locking groove, such that relative positions of the receiving member and the pressing member are positioned.

11. The ultrasound endoscope according to any one of claims 1 to 10,
wherein at least one of the receiving member or the pressing member is formed of metal.

12. The ultrasound endoscope according to any one of claims 1 to 11,
wherein the receiving member and the pressing member are insulated from a ground of the distal end part.

13. The ultrasound endoscope according to any one of claims 1 to 12,
wherein an observation system unit is disposed in the distal end part, and includes an imaging element (90) and a signal cable (94) that is connected to the imaging element, and
the receiving member and the pressing member are insulated from a ground of the signal cable.

14. The ultrasound endoscope according to any one of claims 1 to 13,
wherein the ultrasound transducer and the plurality of signal wire bundles are electrically connected through a substrate (92).

15. The ultrasound endoscope according to claim 14,
wherein the substrate is a flexible print substrate.

## Patentansprüche

1. Ultraschallendoskop, bei dem ein Ultraschallwandler (46) in einem distalen Endteil (40) eines Einführteils (22) angeordnet ist, wobei das Ultraschallendoskop (12) umfasst:
mehrere Signaldrahtbündel (72), die mit dem Ultraschallwandler elektrisch verbunden sind und sich entlang einer Mittelachse des distalen Endteils erstrecken, wobei jedes Signaldrahtbündel mehrere Koaxialkabel (72a), eine Schutzschicht (72b), mit der die mehreren Koaxialkabel beschichtet sind, und eine Außenbeschichtung (72c), mit der die Schutzschicht beschichtet ist, enthält;
ein Empfangselement (110), das auf einer distalen Endseite der mehreren Signaldrahtbündel positioniert ist, wobei das Empfangselement auf einer Außenumfangsseite in dem distalen Endteil entlang eines Innenumfangs des distalen Endteils angeordnet ist und **dadurch gekennzeichnet ist, dass**:
ein Druckelement, das auf der distalen Endseite der mehreren Signaldrahtbündel positioniert ist, wobei das Druckelement auf einer Innenseite in dem distalen Endteil angeordnet ist und mindestens eine Druckfläche umfasst, wobei die mindestens eine Druckfläche in dem distalen Endteil näher an der Mittelachse als das Empfangselement angeordnet ist,
wobei die mehreren Signaldrahtbündel fixiert sind, indem sie zwischen dem Empfangselement und dem Druckelement eingeklemmt werden.

2. Ultraschallendoskop nach Anspruch 1,
wobei in einem Querschnitt von distalem Endteil entlang einer Ebene, die zu der Mittelachse des distalen Endteils (40) senkrecht ist und durch das Empfangselement (110) und das Druckelement (120) verläuft, in einem Fall, in dem ein Bereich von Bereichen, die durch Unterteilen des Querschnitts von distalem Endteil in zwei erhalten werden, wobei eine gerade Linie, die durch die Mittelachse verläuft, als ein erster unterteilter Bereich (AR1) definiert ist und der andere Bereich als ein zweiter unterteilter Bereich (AR2) definiert ist, die mehreren Signaldrahtbündel in dem ersten unterteilten Bereich angeordnet sind.

3. Ultraschallendoskop nach Anspruch 2,
wobei eine Beobachtungssystemeinheit (85) in dem distalen Endteil angeordnet ist und ein Bildgebungselement (90) und ein Signalkabel (94), das mit dem Bildgebungselement verbunden ist, enthält, und
das Signalkabel entlang mindestens eines der mehreren Signaldrahtbündel angeordnet ist.

4. Ultraschallendoskop nach Anspruch 3,
wobei in dem Querschnitt von distalem Endteil die mehreren Signaldrahtbündel und das Signalkabel in dem ersten unterteilten Bereich angeordnet sind.

5. Ultraschallendoskop nach Anspruch 4,
wobei das Druckelement mehrere einzelne Druckflächen (120a) enthält, die die mehreren Signalleiterbündel einzeln für jedes Signalleiterbündel drücken, und jede einzelne Druckfläche eine gekrümmte Form aufweist, die jedem Signalleiterbündel folgt.

6. Ultraschallendoskop nach Anspruch 5,
wobei das Signalkabel zwischen den mehreren einzelnen Druckflächen auf einer von den mehreren Signaldrahtbündeln gegenüberliegenden Seite angeordnet ist, wobei dazwischen das Druckelement eingefügt ist.

7. Ultraschallendoskop nach einem der Ansprüche 2 bis 6, ferner umfassend:
ein Zangenrohr (84), das entlang der Mittelachse in dem distalen Endteil angeordnet ist,
wobei in dem Querschnitt von distalem Endteil die mehreren Signaldrahtbündel in dem ersten unterteilten Bereich angeordnet sind und das Zangenrohr in dem zweiten unterteilten Bereich angeordnet ist.

8. Ultraschallendoskop nach Anspruch 7, wenn er von einem des Anspruchs 3, 4 oder 6 abhängt,
wobei eine Beobachtungssystemeinheit (85) in dem distalen Endteil angeordnet ist und ein Bildgebungselement und ein Signalkabel, das mit dem Bildgebungselement verbunden ist, enthält, und
in dem Querschnitt von distalem Endteil in einem Fall, in dem ein Bereich von Bereichen, die durch Unterteilen des Querschnitts von distalem Endteil in zwei erhalten wird, wobei eine gerade Linie, die durch eine Mittelachse jeweils des Signalkabels und des Zangenrohrs verläuft, als ein dritter unterteilter Bereich (AR3) definiert ist und der andere Bereich als ein vierter unterteilter Bereich (AR4) definiert ist, ein Teil von Signaldrahtbündeln unter den mehreren Signaldrahtbündeln in dem dritten unterteilten Bereich angeordnet ist und der andere Teil von Signaldrahtbündeln in dem vierten unterteilten Bereich angeordnet ist.

9. Ultraschallendoskop nach einem der Ansprüche 1 bis 8,
wobei an einer Position, die durch das Empfangselement und das Druckelement zu fixieren ist, die mehreren Signaldrahtbündel einen freigelegten Bereich, der von der Außenbeschichtung freigelegt ist und ein Isolierelement, das geringere Steifigkeit als die Außenbeschichtung aufweist und mit dem der freigelegte Bereich beschichtet ist, enthalten.

10. Ultraschallendoskop nach einem der Ansprüche 1 bis 9,
wobei eine Verriegelungsnut (110b) mit einem offenen Ende bei einem von dem Empfangselement und dem Druckelement vorgesehen ist, ein Verriegelungsstück (120c), das in die Verriegelungsnut verriegelbar ist, bei dem anderen Element von dem Empfangselement und dem Druckelement vorgesehen ist und das Verriegelungsstück in die Verriegelungsnut verriegelt ist, so dass Relativpositionen des Empfangselements und des Druckelements positioniert sind.

11. Ultraschallendoskop nach einem der Ansprüche 1 bis 10,
wobei mindestens eines von dem Empfangselement und dem Druckelement aus Metall gebildet ist.

12. Ultraschallendoskop nach einem der Ansprüche 1 bis 11,
wobei das Empfangselement und das Druckelement von einem Boden des distalen Endteils isoliert sind.

13. Ultraschallendoskop nach einem der Ansprüche 1 bis 12,
wobei eine Beobachtungssystemeinheit in dem distalen Endteil angeordnet ist und ein Bildgebungselement (90) und ein Signalkabel (94), das mit dem Bildgebungselement verbunden ist, enthält, und
das Empfangselement und das Druckelement von einem Boden des Signalkabels isoliert sind.

14. Ultraschallendoskop nach einem der Ansprüche 1 bis 13,
wobei der Ultraschallwandler und die mehreren Signaldrahtbündel elektrisch über ein Substrat (92) elektrisch verbunden sind.

15. Ultraschallendoskop nach Anspruch 14,
wobei das Substrat ein flexibles Drucksubstrat ist.

## Revendications

1. Endoscope ultrasonore dans lequel un transducteur ultrasonore (46) est disposé dans une partie d'extrémité distale (40) d'une partie d'insertion (22), l'endoscope ultrasonore (12) comprenant :
une pluralité de faisceaux de fils de signal (72) qui sont électriquement connectés au transducteur ultrasonore et s'étendent le long d'un axe central de la partie d'extrémité distale, chaque faisceau de fils de signal comprenant une pluralité de câbles coaxiaux (72a), une couche de blindage (72b) avec laquelle la pluralité de câbles coaxiaux est enrobée, et un revêtement extérieur (72c) avec lequel la couche de blindage est enrobée ;
un élément de réception (110) qui est positionné sur le côté d'extrémité distale de la pluralité de faisceaux de fils de signal, l'élément de réception étant disposé sur un côté périphérique extérieur dans la partie d'extrémité distale le long d'une périphérie intérieure de la partie d'extrémité distale, et **caractérisé par** :
un élément de pression qui est positionné sur le côté d'extrémité distale de la pluralité de faisceaux de fils de signal, l'élément de pression étant disposé sur un côté intérieur dans la partie d'extrémité distale et comprenant au moins une surface de pression, dans lequel au moins une surface de pression est disposée plus près de l'axe central que l'élément de réception dans la partie d'extrémité distale,
dans lequel la pluralité de faisceaux de fils de signal sont fixés en étant pris en sandwich entre l'élément de réception et l'élément de pression.

2. Endoscope ultrasonore selon la revendication 1,
dans lequel dans une section transversale de partie d'extrémité distale prise le long d'un plan qui est perpendiculaire à l'axe central de la partie d'extrémité distale (40) et passe par l'élément de réception (110) et l'élément de pression (120), dans un cas où une zone de zones obtenues en divisant la section transversale de partie d'extrémité distale en deux, une ligne droite passant par l'axe central étant définie comme une première zone divisée (AR1) et l'autre zone étant définie comme une deuxième zone divisée (AR2), la pluralité de faisceaux de fils de signal est disposée dans la première zone divisée.

3. Endoscope ultrasonore selon la revendication 2,
dans lequel une unité de système d'observation (85) est disposée dans la partie d'extrémité distale et comprend un élément d'imagerie (90) et un câble de signal (94) qui est connecté à l'élément d'imagerie, et
le câble de signal est disposé le long d'au moins un de la pluralité de faisceaux de fils de signal.

4. Endoscope ultrasonore selon la revendication 3,
dans lequel dans la section transversale de partie d'extrémité distale, la pluralité de faisceaux de fils de signal et le câble de signal sont disposés dans la première zone divisée.

5. Endoscope ultrasonore selon la revendication 4,
dans lequel l'élément de pression comprend une pluralité de surfaces de pression individuelles (120a) qui pressent individuellement la pluralité de faisceaux de fils de signal pour chaque faisceau de fils de signal, et chaque surface de pression individuelle a une forme courbée suivant chaque faisceau de fils de signal.

6. Endoscope ultrasonore selon la revendication 5,
dans lequel le câble de signal est disposé entre la pluralité de surfaces de pression individuelles sur un côté opposé à la pluralité de faisceaux de fils de signal, dans lequel l'élément de pression est interposé entre eux.

7. Endoscope ultrasonore selon l'une quelconque des revendications 2 à 6, comprenant en outre :
un tube de forceps (84) qui est disposé dans la partie d'extrémité distale le long de l'axe central,
dans lequel dans la section transversale de partie d'extrémité distale, la pluralité de faisceaux de fils de signal sont disposés dans la première zone divisée et le tube de forceps est disposé dans la deuxième zone divisée.

8. Endoscope ultrasonore selon la revendication 7, lorsqu'il dépend de l'une quelconque des revendications 3, 4 ou 6,
dans lequel une unité de système d'observation (85) est disposée dans la partie d'extrémité distale et comprend un élément d'imagerie et un câble de signal qui est connecté à l'élément d'imagerie, et
dans la section transversale de partie l'extrémité distale, dans un cas où une zone de zones obtenues en divisant la section transversale de partie d'extrémité distale en deux, une ligne droite passant par un axe central de chacun du câble de signal et du tube de forceps étant définie comme une troisième zone divisée (AR3) et l'autre zone étant définie comme une quatrième zone divisée (AR4), une partie des faisceaux de fils de signal parmi la pluralité de faisceaux de fils de signal est disposée dans la troisième zone divisée et l'autre partie des faisceaux de fils de signal est disposée dans la quatrième zone divisée.

9. Endoscope ultrasonore selon l'une quelconque des revendications 1 à 8,
dans lequel à une position à fixer par l'élément de réception et l'élément de pression, la pluralité de faisceaux de fils de signal comprend une zone exposée qui est exposée du revêtement extérieur, et un élément isolant qui a une rigidité inférieure à celle du revêtement extérieur et avec lequel la zone exposée est revêtue.

10. Endoscope ultrasonore selon l'une quelconque des revendications 1 à 9,
dans lequel une rainure de verrouillage (110b) avec une extrémité ouverte est prévue dans l'un de l'élément de réception et l'élément de pression, une pièce de verrouillage (120c) pouvant être verrouillée dans la rainure de verrouillage est prévue dans l'autre élément de l'élément de réception et l'élément de pression, et la pièce de verrouillage est verrouillée dans la rainure de verrouillage, de sorte que des positions relatives de l'élément de réception et de l'élément de pression sont positionnées.

11. Endoscope ultrasonore selon l'une quelconque des revendications 1 à 10,
dans lequel au moins l'un de l'élément de réception et de l'élément de pression est formé de métal.

12. Endoscope ultrasonore selon l'une quelconque des revendications 1 à 11,
dans lequel l'élément de réception et l'élément de pression sont isolés d'une masse de la partie d'extrémité distale.

13. Endoscope ultrasonore selon l'une quelconque des revendications 1 à 12,
dans lequel une unité de système d'observation est disposée dans la partie d'extrémité distale et comprend un élément d'imagerie (90) et un câble de signal (94) qui est connecté à l'élément d'imagerie, et
l'élément de réception et l'élément de pression sont isolés d'une masse du câble de signal.

14. Endoscope ultrasonore selon l'une quelconque des revendications 1 à 13,
dans lequel le transducteur ultrasonore et la pluralité de faisceaux de fils de signal sont connectés électriquement via un substrat (92).

15. Endoscope ultrasonore selon la revendication 14,
dans lequel le substrat est un substrat d'impression flexible.
